(19) Europäisches Patentamt
European Patent Office
Office européen des brevets

(11) Veröffentlichungsnummer: **0 038 984**
**B1**

(12) # EUROPÄISCHE PATENTSCHRIFT

(45) Veröffentlichungstag der Patentschrift: **03.07.85**

(51) Int. Cl.⁴: **C 07 C 69/96**, C 07 C 68/00, B 01 J 31/26

(21) Anmeldenummer: **81102792.9**

(22) Anmeldetag: **11.04.81**

(54) **Verfahren zur Herstellung von Kohlensäureestern.**

(30) Priorität: **26.04.80 DE 3016187**

(43) Veröffentlichungstag der Anmeldung: **04.11.81 Patentblatt 81/44**

(45) Bekanntmachung des Hinweises auf die Patenterteilung: **03.07.85 Patentblatt 85/27**

(84) Benannte Vertragsstaaten: **DE FR GB IT NL**

(56) Entgegenhaltungen:
**DE - A - 2 110 194**
**DE - A - 2 334 736**

(73) Patentinhaber: **BAYER AG, Konzernverwaltung RP Patentabteilung, D-5090 Leverkusen 1 Bayerwerk (DE)**

(72) Erfinder: **Stammann, Günter, Dr., Silesiusstrasse 78, D-5000 Köln (DE)**
Erfinder: **Becker, Robert, Dr., Fichtestrasse 2a, D-5090 Leverkusen 1 (DE)**
Erfinder: **Grolig, Johann, Dr., Heinrich-Lübke-Strasse 22, D-5090 Leverkusen (DE)**
Erfinder: **Waldmann, Helmut, Dr., Henry-T.-von Böttinger-Strasse 15, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 038 984 B1

**Beschreibung**

Die vorliegende Erfindung betrifft die Herstellung von Kohlensäureestern (Carbonaten) durch Umsetzung von Alkoholen mit Gemischen aus molekularem Sauerstoff und Kohlenmonoxid in flüssiger Phase in Gegenwart eines Katalysatorsystems, welches Kupfer, chemisch gebundenen Sauerstoff, chemisch gebundenes Halogen und mindestens eine Stickstoffbase enthält.

Es ist bereits bekannt, dass Kohlensäureester aus Alkoholen oder Phenolen mit Phosgen unter Verwendung stöchiometrischer Mengen Base hergestellt werden können. Unter Vermeidung des schwierig zu handhabenden Phosgens können Kohlensäureester auch aus Hydroxylgruppen enthaltenden organischen Verbindungen und Kohlenmonoxid unter oxidierenden Bedingungen in Gegenwart bestimmter Katalysatoren hergestellt werden, z. B. nach folgender Reaktionsgleichung:

$$2\ ROH + CO + 1/2\ O_2 \underset{Kat.}{\rightarrow} (RO)_2CO + H_2O$$

So wird in US-PS 3 846 468 und US-PS RE 29 338 angegeben, dass mit stöchiometrischen Katalysatormengen Kohlensäureester nach obiger Reaktionsgleichung im Temperaturbereich von $-20\,°C$ bis $+110\,°C$ erhalten werden können, wobei der Katalysator vorzugsweise aus dem Gemisch eines Kupferhalogenids mit einer organischen Stickstoffbase besteht. Aufgrund der niedrigen Reaktionstemperatur ist jedoch bei diesem Verfahren das Abführen der Reaktionswärme und deren Nutzung technisch schwierig und aufwendig und die erzielbaren Raum-Zeit-Ausbeuten an organischem Carbonat sind gering.

In der DE-OS 2 743 690 wird ebenfalls ein derartiges Verfahren beschrieben, bei dem vorzugsweise Kupfersalze als Katalysatoren angewendet werden. Wie aus den Beispielen dieser DE-OS hervorgeht, sind erhebliche Mengen an Katalysator für dieses Verfahren notwendig. Dadurch ergeben sich bei diesem Verfahren technische Probleme der Katalysatoreinbringung, Katalysatorabtrennung und Katalysatorrückführung. Bei diesen hohen Katalysatorkonzentrationen muss man bei relativ niedrigen Temperaturen arbeiten, um hohe Selektivitäten zu erreichen und um Korrosionen vermeiden zu können.

Es wurde nun ein Verfahren zur Herstellung von Kohlensäureestern aus Alkoholen, Kohlenmonoxid und molekularem Sauerstoff in flüssiger Phase gefunden, welches dadurch gekennzeichnet ist, dass man in Gegenwart eines Katalysatorsystems arbeitet, welches

a) Kupfer und/oder Kupferionen,
b) Sauerstoffanionen und/oder Sauerstoff enthaltende Anionen,
c) Halogenidionen,
d) eine oder mehrere Stickstoffbasen,
e) gegebenenfalls weitere Metallionen von Metallen der zweiten Hauptgruppe, aus den Gruppen der Lanthaniden und/oder Aktiniden und/oder von Metallen mit Ordnungszahlen von 25 bis 30

enthält.

Als Ausgangsprodukte für das erfindungsgemässe Verfahren werden einerseits Kohlenmonoxid und molekularer Sauerstoff eingesetzt. Sauerstoff kann in reiner Form oder in Form von Gemischen mit Inertgasen, wie Stickstoff, insbesondere in Form von Luft, verwendet werden. Kohlenmonoxid und Sauerstoff bzw. Sauerstoff enthaltende Gasgemische können in beliebiger Reihenfolge oder gemischt eingesetzt werden, wobei darauf zu achten ist, dass man ausserhalb der Explosionsgrenzen arbeitet.

Als Ausgangsprodukte einsetzbare Alkohole können von der verschiedensten Art sein. So können beispielsweise lineare oder verzweigte Alkanole, Cycloalkanole, Alkenole, Cycloalkenole, Aralkylalkohole und ähnliche eingesetzt werden, die ein- oder mehrwertig sein und 1 bis 20 C-Atome enthalten können. Die Alkohole können Substituenten enthalten, wie Sauerstoff, Stickstoff, Schwefel oder Halogen, beispielsweise eine oder mehrere Halogen-, Sulfoxid-, Sulfon-, Amin-, Amid-, Carbonyl- und/oder Carbonsäureestergruppen. Vorzugsweise wird in das erfindungsgemässe Verfahren einer der folgenden Alkohole eingesetzt: Methylalkohol, Ethylalkohol, Propanol, Isopropanol, Butanol, Pentanol, Hexanol, Cyclohexanol, Benzylalkohol, Ethylenglykol, Diethylenglykol, Propylenglykol, Dipropylenglykol, Glycerin, Hexantriol und ähnliche, sowie höherfunktionelle Polyole. Besonders bevorzugt werden einwertige aliphatische Alkohole mit 1 bis 6 C-Atomen eingesetzt. Ganz besonders bevorzugt sind dabei Methanol, Ethanol, 1-Propanol und 2-Propanol.

Im allgemeinen werden die Alkohole in das erfindungsgemässe Verfahren im Überschuss, d. h. bezogen auf das Katalysatorsystem, in einem Gewichtsverhältnis von grösser als 1 : 1 eingesetzt. Vorzugsweise werden die Alkohole in das erfindungsgemässe Verfahren in solchen Mengen eingesetzt, dass sie zum Katalysatorsystem im Gewichtsverhältnis 5 : 1 bis 300 : 1 stehen. Bevorzugt werden die Alkohole im Überschuss in Bezug auf Kohlenmonoxid eingesetzt, wobei der nicht umgesetzte überschüssige Alkohol als Reaktionsmedium dient. Der molekulare Sauerstoff kann bezüglich des Alkohols im Unterschuss oder im Überschuss eingesetzt werden. Bevorzugt wird ein Inertgas, wie Stickstoff, zugegeben oder es wird mit Luft gearbeitet, wobei die Mengen so bemessen werden, dass man ausserhalb des Explosionsbereiches der Sauerstoff-Kohlenmonoxid-Gemische bzw. der Sauerstoff-Alkohol-Gemische arbeiten kann. Ohne Inertgaszugabe wird die Sauerstoffzugabe so bemessen, dass explosible Gemische mit Kohlenmonoxid und der Alkoholkomponente vermieden werden. Bevorzugt wird der molekulare Sauerstoff in Form von Luft oder Luft-Stickstoff-Gemischen eingesetzt.

Das erfindungsgemäss zu verwendende Katalysatorsystem enthält:

a) Kupfer und/oder Kupferionen,
b) Sauerstoffanionen und/oder Sauerstoff enthaltende Anionen,
c) Halogenidionen,
d) eine oder mehrere Stickstoffbasen,
e) gegebenenfalls weitere Metallionen von Metallen der zweiten Hauptgruppe, aus den Gruppen der Lanthaniden und/oder Aktiniden und/oder von Metallen mit den Ordnungszahlen von 25 bis 30.

Die im erfindungsgemäss zu verwendenden Katalysatorsystem enthaltene Komponente a) kann als Kupfer in jeder bekannten Oxidationsstufe eingesetzt werden. Unter den Reaktionsbedingungen können daraus katalytisch wirksame Kupferspezies entstehen, die in 0- bis +3-wertiger Form vorliegen können. Im Hinblick auf eine optimale Selektivität der Kohlensäureesterbildung ist es vorteilhaft, wenn Kupfer überwiegend in der Oxidationsstufe +2 in die Reaktion eingebracht wird.

Das erfindungsgemäss zu verwendende Katalysatorsystem enthält bevorzugt ionische Kupferverbindungen. Bevorzugt ist dabei das kationische Kupfer mit einem oder mehreren Oxy-, Hydroxy- oder anderen sauerstoffhaltigen Anionen kombiniert. Beispiele für erfindungsgemäss einsetzbare Kupferverbindungen sind Kupfer-(II)-oxid, Kupfer-(I)-oxid, Kupfer-(II)-hydroxid, Kupfer-(II)-nitrat, Kupfer-(II)-hydroxycarbonat, Kupfer-(II)-hydroxynitrat, Kupfer-(II)-acetat, Kupfer-(II)-oxalat, Kupfer-(II)-citrat, Kupfer-(II)-acetylacetonat, Kupfer-(II)-ethylat und Kupfernaphthenat, wobei auch mehrere Kupferverbindungen eingesetzt werden können. Besonders vorteilhaft werden in das erfindungsgemäss zu verwendende Katalysatorsystem Kupfer-(II)-oxid, Kupfer-(II)-hydroxycarbonat oder Kupfer-(II)-hydroxid alleine oder in beliebigen Gemischen untereinander eingesetzt. Bei Verwendung einer oder mehrerer der vorgenannten Verbindungen können so die Katalysatorkomponenten a) und b) eingebracht werden.

Kupferverbindungen, die sowohl Sauerstoff als auch Halogen enthalten, sind als Katalysatorkomponenten ebenfalls geeignet. Bevorzugt ist hierbei Kupfer-(II)-oxychlorid und Atakamit ($Cu_2(OH)_3Cl$). Bei Verwendung solcher Verbindungen als Komponenten des erfindungsgemäss zu verwendenden Katalysatorsystems kann von dem Zusatz weiterer Kupferionen oder Metallionen entsprechend e) abgesehen werden. Die vorgenannten Verbindungen können die Katalysatorkomponenten a), b) und c) einbringen.

Die in dem erfindungsgemäss zu verwendenden Katalysatorsystem enthaltene Komponente c) können die Halogenidionen Fluorid, Chlorid, Bromid oder Jodid allein oder in Kombination miteinander sein. Im allgemeinen ist es vorteilhaft, als Halogenidion Chlorid als Katalysatorkomponente c) zu verwenden. Hierzu kann Chlorid beispielsweise in Form von Metallchloriden, Metalloxychloriden, Metallhydroxychloriden, aber auch in Form von Ammoniumchloriden eingesetzt werden. Als Gegenionen des Chloridions können Kupferionen und/oder Metallionen entsprechend der Katalysatorkomponente e) in stabilen Wertigkeitsstufen gewählt werden oder Kombinationen davon. Bevorzugt werden $Cu^{2+}$, $Fe^{2+}$, $Fe^{3+}$, $Co^{2+}$, $Ni^{2+}$, $Ca^{2+}$, $Mg^{2+}$ und Kationen der Lanthanidengruppe verwendet. Sofern als Metallion hier kein Kupfer verwendet wird, handelt es sich um die im erfindungsgemäss zu verwendenden Katalysatorsystem gegebenenfalls enthaltenen weiteren Metallionen entsprechend Komponente e).

Es ist vorteilhaft, einen Teil des Chlorides in Form eines Ammoniumchlorides, vorzugsweise als Hydrochlorid einer Stickstoffbase und besonders bevorzugt als Hydrochlorid einer organischen Stickstoffbase einzusetzen und den Rest als Chlorid mit den Gegenionen entsprechend Katalysatorkomponente a) und/oder e).

Als Katalysatorkomponente d) geeignete Stickstoffbasen umfassen Ammoniak, ein- oder mehrwertige primäre, sekundäre und tertiäre Alkyl-, Cycloalkyl-, Aryl- und Aralkylamine, wobei Stickstoff selbst als Teil eines aliphatischen oder aromatischen Ringsystems oder als Brückenatom in einem cyclischen oder polycyclischen Ringsystem gebunden vorliegen kann. Beispielsweise können derartige Stickstoffbasen 1 bis 700 C-Atome enthalten.

Auch substituierte Stickstoffbasen können geeignet sein. Als Beispiele für geeignete organische Stickstoffbasen seien genannt: n-Butylamin, Diethylamin, Triethylamin, Hexamethylendiamin, Dicyclohexylamin, Anilin, 2,4-Diaminotoluol, 3-Aminopropylethylether, 2-Methylpiperidin, 1-Amino-2-methylindolin, Pyridin, 2,4,6-Trimethylpyridin, Chinolin, 6-Chlor-2-methylchinolin, 3,6-Dinitro-9-H-carbazol, 4-Phenylmorpholin, 1H-Phenothiazin, 1,4-Diazabicyclo[2.2.2]octan, 1-Aza-4,6-dioxa-bicyclo[3.3.0]octan, wobei bevorzugt aliphatische Amine und Stickstoff enthaltende Heteroaromaten, wie Pyridin, eingesetzt werden. Geeignete Stickstoffbasen sind auch Benzimidazol, Benztriazol, 2-(5-Aminopentyl)-benzimidazol und 1,2-Pentamethylen-benzimidazol. Auf die vorstehende Art und Weise kann die Katalysatorkomponente d) eingebracht werden.

Für die Durchführung des erfindungsgemässen Verfahrens ist es vorteilhaft, eine oder mehrere der Stickstoffbasen als Hydrochloride oder komplexiert mit den Katalysatorkomponenten a) und/oder gegebenenfalls e) einzusetzen. Beispielsweise sind hierfür geeignet: Pyridinhydrochlorid, Dipyridino-kupfer-(II)-hydroxychlorid, Dipyridino-kupfer-(II)-chlorid, Dipyridino-kobalt-(II)-chlorid und Ammoniumtetrachloro-(II)-cuprat. Sofern eine derartige Verbindung eingesetzt wird, ist es möglich, mit einer Verbindung die Katalysatorkomponenten a), c) und d) einzubringen, sowie gegebenenfalls auch b) und e).

Für das erfindungsgemässe Verfahren ist es vorteilhaft, die Katalysatorkomponenten a) bis d) und gegebenenfalls e) in bestimmten Verhältnissen zueinander einzusetzen, um eine optimale Aktivität und Selektivität des Katalysatorsystems zu erzielen.

Der Halogenidgehalt des erfindungsgemäss zu verwendenden Katalysatorsystems wird vorzugsweise so eingestellt, beispielsweise durch geeignete Kombination der einzelnen Katalysatorkomponenten, dass das Verhältnis von Halogenid zu Gesamtmetall, ausgedrückt in Gramm-Atom, 0,5 : 1 bis 2 : 1, insbesondere 0,8 : 1 bis 1,4 : 1 beträgt. Chloridanteile, die in Form eines Hydrochlorides und bevorzugt in Form des Pyridinhydrochlorides eingebracht werden, werden vorzugsweise so gewählt, dass das Verhältnis dieses Chloridanteils zum Gesamtmetall, ausgedrückt in Grammatom, 0,1 : 1 bis 1 : 1, vorzugsweise 0,3 : 1 bis 0,8 : 1, beträgt.

Die Stickstoffbase (Katalysatorkomponente d)) wird vorteilhaft in einer solchen Menge eingesetzt, dass das molare Verhältnis der Stickstoffbase zum Gesamtmetall des Katalysatorsystems 0,1 : 1 bis 1,4 : 1, vorzugsweise 0,3 : 1 bis 1,2 : 1, beträgt. Dieses Verhältnis ist unabhängig davon, ob die Stickstoffbase in freier Form oder komplexiert mit einem der geeigneten Metallsalze oder als Ammoniumchlorid bzw. Hydrochlorid eingesetzt wird.

Die oxidische oder Sauerstoff enthaltende Katalysatorkomponente entsprechend b) stellt eine wesentliche Komponente des Katalysatorsystems dar. Sie wird bevorzugt als Kupferverbindung eingebracht. Die Katalysatorkomponente b) wird bezüglich der übrigen, als Metallhalogenide oder als Metallhalogenidkomplexe vorliegenden Metallverbindungen vorzugsweise im molaren Verhältnis von 0,5 : 1 bis 3 : 1, vorzugsweise 1,5 : 1 bis 2,2 : 1 eingesetzt. Verwendet man hierfür Oxyhalogen- oder Hydroxyhalogenverbindungen des Kupfers so können mit diesen Verbindungen die Katalysatorkomponenten a) bis c) eingebracht werden. Der Zusatz weiterer Kupferionen und/oder weiterer Metallionen entsprechend e) ist dann zwar möglich, jedoch nicht zwingend erforderlich.

Von den vielfältigen Möglichkeiten zur Herstellung eines erfindungsgemäss verwendbaren Katalysatorsystems aus den oben angeführten Komponenten sind einige Kombinationen für das erfindungsgemässe Verfahren besonders vorteilhaft, wenn die Zusammensetzung entsprechend den oben angeführten Verhältnissen gewählt werden soll. Zu diesen bevorzugten Kombinationen zählen folgende:

$CuCO_3 \cdot Cu(OH)_2$, $CuCl_2$ und Pyridinhydrochlorid;
$CuCO_3 \cdot Cu(OH)_2$, $CoCl_2$ und Pyridinhydrochlorid;
$Cu_2OCl_2$ und Pyridinhydrochlorid;
$Cu_2(OH)_3Cl$ und Pyridinhydrochlorid;
$Cu(OH)Cl \cdot 2$ Pyridin, $CuCl_2 \cdot 2$ Pyridin,
$CuCl_2 \cdot 2H_2O$ und $Cu_2(OH)_3Cl$.

Selbstverständlich sind auch Gemische der angeführten besonders vorteilhaften Kombinationen für das erfindungsgemässe Verfahren geeignet.

Ein besonderer Vorteil des erfindungsgemäss zu verwendenden Katalysatorsystems besteht darin, dass das Katalysatorgemisch nach dem Entspannen und Abkühlen der Reaktionsmasse ausfällt, gegebenenfalls nach teilweisem Abtreiben des Lösungsmittels oder des überschüssigen Alkohols. Das ausgefallene Katalysatorgemisch ist rückführbar und noch katalytisch aktiv. Werden als metallische Komponenten ausschliesslich Kupferverbindungen in dem erfindungsgemässen Katalysatorsystem eingesetzt, so kann das Katalysatorgemisch nach der Reaktion, abhängig von der Konzentration der Einsatzkomponenten, wechselnde Mengen von $CuCl_2 \cdot 2H_2O$, $Cu_2(OH)_3Cl$ (Atakamit), $CuCl_2 \cdot 2$ Pyridin und $Cu(OH)Cl \cdot 2$ Pyridin als Hauptbestandteile enthalten.

Wird als Stickstoffbase Pyridin oder Pyridinhydrochlorid in den als bevorzugt angegebenen Mengen zugegeben, so enthält die Produktlösung gegebenenfalls Pyridin, aber allenfalls nur in Spuren. Die destillative Aufarbeitung des Produktes nach der Katalysatorabtrennung wird dadurch normalerweise nicht gestört. Das eingesetzte Pyridin liegt in diesem Fall nahezu ausschliesslich komplex gebunden an Kupferverbindungen oder gegebenenfalls an Metallverbindungen entsprechend der Katalysatorkomponente e) vor und ist auch in dieser Form ein katalytisch wirksamer Katalysatorbestandteil.

Es ist im allgemeinen vorteilhaft, vor der Rückführung des Katalysators einen Anteil von beispielsweise 5 bis 30 Gew.-%, vorzugsweise 10 bis 15 Gew.-%, zu entnehmen und das darin chemisch und/oder physikalisch gebundene Reaktionswasser zu entfernen, z.B. durch eine thermische Behandlung. Entsprechend der entnommenen Katalysatormenge wird dann vorzugsweise ein frischer Anteil oder ein z.B. durch die thermische Behandlung regenerierter Anteil des erfindungsgemäss zu verwendenden Katalysatorsystems dem rückgeführten Katalysator zugegeben.

Das erfindungsgemäss zu verwendende Katalysatorsystem ermöglicht die Herstellung von Kohlensäureestern in hoher Selektivität und unter Reaktionsbedingungen, die hohe Raum-Zeit-Ausbeuten und gegebenenfalls die technische Nutzung der Reaktionswärme aus der stark exothermen Reaktion zulassen. Beispielsweise kann beim erfindungsgemässen Verfahren die auf relativ hohem Temperaturniveau anfallende Reaktionswärme dazu benutzt werden, um das Lösungsmittel bzw. den überschüssigen Alkohol abzudestillieren.

Bezogen auf das gesamte Einsatzmaterial, einschliesslich Lösungsmittel, beträgt die gesamte Katalysatormenge beim erfindungsgemässen Verfahren vorzugsweise 0,5 bis 8 Gew.-%.

Besonders vorteilhaft beim erfindungsgemässen Verfahren ist es, dass auch mit sehr kleinen Mengen Metallverbindungen entsprechend den Komponenten a) und/oder gegebenenfalls e) hohe Raum/Zeit-Ausbeuten und Selektivitäten erzielt werden können. Es ist überraschend, dass mit dem erfindungsgemäss zu verwendenden Katalysatorsystem nicht die bei Kupferhalogeniden in Verbindung mit gebräuchlichen Reaktormaterialien bekannten Korrosionsprobleme auftreten. Für das erfindungsgemässe Verfahren geeignete Werkstoffe sind beispielsweise solche entsprechend DIN 17 440 mit den Bezeichnungen 1.4571, 1.4577 und 1.4439, sowie Legierungen auf Nickel-Basis, beispielsweise der Werkstoff mit der Bezeichnung Ni Mo 16 Cr 16 Ti.

Das erfindungsgemässe Verfahren zur Herstellung von Kohlensäureestern kann diskontinuierlich oder kontinuierlich durchgeführt werden.

Man kann die Reaktion in der Weise durchführen, dass man die Alkohol-Komponente mit dem erfindungsgemäss zu verwendenden Katalysatorsystem in einem ersten Reaktionsschritt zunächst mit Kohlenmonoxid umsetzt und in einem zweiten Reaktionsschritt mit molekularem Sauerstoff oxidiert. Bei dieser Arbeitsweise wird der Kohlensäureester maximal in stöchiometrischen Mengen bezüglich des Metalles im erfindungsgemässen Katalysatorsystem gebildet.

Die erfindungsgemässe Umsetzung kann in Gegenwart oder in Abwesenheit eines Lösungsmittels durchgeführt werden. Im allgemeinen dient der vorzugsweise im Überschuss eingesetzte Alkohol als Lösungsmittel. Die Mitverwendung inerter Lösungsmittel, die bis zu 80 Gew.-% des gesamten Reaktionsansatzes ausmachen können, ist jedoch auch möglich.

Brauchbare Lösungsmittel sind unter den Reaktionsbedingungen gegen die Reaktionskomponenten und das Katalysatorsystem inerte Lösungsmittel, beispielsweise aromatische, cycloaliphatische und aliphatische Kohlenwasserstoffe, die vorzugsweise durch Halogen substituiert sind, wie Chlorbenzol, Dichlorbenzol, Trichlorbenzol, Chlornaphthalin, Chlorcyclohexan, Methylenchlorid, Tetrachlorkohlenstoff, Tetrachlorethan, Trichlorfluorethan und ähnliche Verbindungen.

Die Reaktionstemperatur für das erfindungsgemässe Verfahren kann im allgemeinen zwischen 50 und etwa 250 °C liegen. Vorzugsweise wird zwischen 110 und 220 °C und besonders vorteilhaft im Bereich von 140 bis 200 °C gearbeitet. Der Druck muss so bemessen sein, dass stets das Vorliegen einer flüssigen Phase gewährleistet ist. Er kann bei Reaktionstemperatur im allgemeinen weniger als 250 bar betragen, vorzugsweise arbeitet man im Bereich zwischen 50 und 200 bar.

Das erfindungsgemässe Verfahren wird durch die nachstehenden Beispiele weiter erläutert, ohne sich darauf zu beschränken.

Beispiele

Beispiele 1–8

(Beispiele 1 und 2 sind Vergleichsbeispiele zu den Beispielen 3 bis 8, da die Beispiele 1 und 2 keine Katalysatorkomponente entsprechend b) enthalten)

In einem 0,7 l Edelstahlautoklaven wurden jeweils 96,86 bis 99,0 Gew.-% Ethanol (200 g) und die Katalysatorkomponenten entsprechend Tabelle 1 vorgelegt. Nach Spülung mit Stickstoff wurden bei Raumtemperatur zunächst 100 bar Kohlenmonoxid, danach 25 bar Luft aufgepresst. Unter Rühren wurde das Reaktionsgemisch 1 Stunde auf 180 °C erhitzt. Nach dem Abkühlen wurde entspannt und die Reaktionsmasse mit frisch aufgepresstem Kohlenmonoxid/Luft-Gemisch ein zweites Mal in gleicher Weise zur Reaktion gebracht. Nach erneutem Abkühlen und Entspannen wurde die Reaktionslösung jeweils quantitativ gaschromatographisch analysiert. Proben der Produktgase enthielten durchschnittlich 1 bis 2 Vol.-% $CO_2$.

Tabelle 1

| Beispiel-Nr. | Katalysator (Gew.-%) (Py = Pyridin) | Umsatz (%) (bezogen auf Ethanol) | Diethylcarbonat Selektivität (Mol-%) | g Ausbeute pro g Katalysator |
|---|---|---|---|---|
| 1 | $CuCl_2$ (1,94) Py (1,20) | 11,3 | 92,7 | 4,2 |
| 2 | $Cu_2Cl_2$ (1,47) Py (0,58) | 7,6 | 98,0 | 4,5 |
| 3 | CuO (0,97), $CuCl_2$ (0,97), Py · HCl (0,97) | 12,2 | 98,5 | 5,1 |
| 4 | $Cu_2OCl_2$ (1,94), Py · HCl (0,97) | 11,7 | 98,0 | 4,9 |
| 5 | Atakamit (1,37), Py · HCl (0,49) | 9,1 | 97,0 | 5,9 |
| 6 | $CuCl_2$ · Py (0,1), Atakamit (0,3) Cu(OH)Cl · 2 Py (0,6) | 6,4 | 99,1 | 8,1 |
| 7 | $CuCl_2$ · 2 Py (0,7), Atakamit (0,7), Py (0,1) | 6,7 | 96,0 | 5,5 |
| 8 | $(NH_4)_2CuCl_4$ (1,0), CuO (1,0) | 13,6 | 85,0 | 7,4 |

Beispiel 9

In einem 0,7 l Edelstahlautoklaven wurde ein Gemisch von 97,1 Gew.-% (200 g) Ethanol und jeweils 0,97 Gew.-% der Katalysatorkomponenten Kupfer-(II)-oxid, Kupfer-(II)-chlorid (wasserfrei) und Pyridinhydrochlorid zweimal mit Gasgemischen aus 100 bar Kohlenmonoxid und 20 bar Luft je 2 Stunden bei 120 °C zur Reaktion gebracht. Ethanol wurde zu 7,6% bei 99,9% Selektivität zu Diethylcarbonat umgesetzt.

Beispiel 10

Es wurde wie in Beispiel 9 gearbeitet, jedoch bestand das Katalysatorsystem aus 0,54 Gew.-% Kupfer-(II)-oxid, 0,88 Gew.-% Kupfer-(II)-chlorid (wasserfrei) und 1,07 Gew.-% Anilinhydrochlorid.

Ethanol wurde zu 5,8% bei 99,6% Selektivität zu Diethylcarbonat umgesetzt.

Beispiele 11–19

Es wurde wie in Beispiel 1 angegeben gearbeitet, jedoch bestand das Katalysatorsystem aus 0,97 Gew.-% Kupfer-(II)-oxid, 0,97 Gew.-% Pyridinhydrochlorid und aus 0,68 bis 1,20 Gew.-% eines Metallchlorids. Die dabei erhaltenen Ergebnisse sind aus Tabelle 2 ersichtlich.

Tabelle 2

| Beispiel-Nr. | Metallchlorid (Gew.-%) | Umsatz (%) (bezogen auf Ethanol) | Selektivität (%) Diethylcarbonat (bezogen auf Ethanol) |
|---|---|---|---|
| 11 | $MgCl_2$ (0,68) | 7,2 | 98,9 |
| 12 | $CaCl_2$ (0,83) | 8,9 | 97,9 |
| 13 | $LaCl_3$ (1,20) | 8,8 | 98,5 |
| 14 | $MnCl_2$ (0,92) | 7,6 | 97,7 |
| 15 | $FeCl_2$ (0,97) | 10,2 | 94,9 |
| 16 | $FeCl_3$ (0,78) | 11,1 | 90,7 |
| 17 | $CoCl_2$ (0,97) | 9,6 | 99,3 |
| 18 | $NiCl_2$ (0,97) | 9,4 | 98,0 |
| 19 | $ZnCl_2$ (0,97) | 6,7 | 98,2 |

Beispiele 20–23

Diese Beispiele belegen, dass mit dem erfindungsgemäss zu verwendenden Katalysatorsystem durch Erhöhung des Sauerstoffangebotes zunehmende Carbonatausbeuten erhalten werden.

In einem 0,7 l Edelstahlautoklaven wurde ein Gemisch von 97,1 Gew.-% (200 g) Ethanol und jeweils 0,97 Gew.-% der Katalysatorkomponenten Kupfer-(II)-oxid, Kupfer-(II)-chlorid (wasserfrei) und Pyridinhydrochlorid mit Gasgemischen aus 100 bar Kohlenmonoxid und 20 bar Luft jeweils 2 Stunden bei 180 °C in 1 bis 5 Reaktionszyklen zur Reaktion gebracht. Die Ergebnisse sind aus Tabelle 3 ersichtlich.

Tabelle 3

| Beispiel-Nr. | Zahl der Reaktionszyklen | Ausbeute an Diethylcarbonat (% der Theorie) |
|---|---|---|
| 20 | 1 | 9,3 |
| 21 | 2 | 12,0 |
| 22 | 3 | 16,8 |
| 23 | 5 | 25,1 |

Beispiele 24–26

Es wurde wie im Beispiel 3 gearbeitet, jedoch wurden anstelle von Ethanol jeweils die Alkohole Methanol, 1-Propanol und 2-Propanol eingesetzt. Die dabei erhaltenen Ergebnisse sind aus Tabelle 4 ersichtlich.

Tabelle 4

| Beispiel-Nr. | Alkohol | Ausbeute an Dialkylcarbonat (% der Theorie) |
|---|---|---|
| 24 | Methanol | 7,7 |
| 25 | 1-Propanol | 10,6 |
| 26 | 2-Propanol | 1,9 |

Beispiele 27 und 28

In einem 0,7 l Edelstahlautoklaven wurden 97,1 Gew.-% (200 g) Ethanol mit einem Katalysatorgemisch bestehend aus 0,97 Gew.-% Kupfer-(II)-chlorid, 0,97 Gew.-% Pyridinhydrochlorid und 0,97 Gew.-% einer weiteren Komponente zweimal mit Gasgemischen aus 100 bar Kohlenmonoxid und 20 bar Luft jeweils 1 Stunde bei 180 °C zur Reaktion gebracht. Die Ergebnisse sind aus Tabelle 5 ersichtlich.

Tabelle 5

| Beispiel-Nr. | zusätzliche Komponente | Ausbeute an Diethylcarbonat (%), bezogen auf Ethanol |
|---|---|---|
| 27 | Kupfer-(II)-acetat-hydrat | 8,0 |
| 28 | Kupfer-(II)-hydroxidcarbonat | 9,8 |

**Patentansprüche**

1. Verfahren zur Herstellung von Kohlensäureestern aus Alkoholen, Kohlenmonoxid und molekularem Sauerstoff in flüssiger Phase, dadurch gekennzeichnet, dass man in Gegenwart eines Katalysatorsystems arbeitet, welches

a) Kupfer und/oder Kupferionen,
b) Sauerstoffanionen und/oder Sauerstoff enthaltende Anionen,
c) Halogenidionen,
d) eine oder mehrere Stickstoffbasen,
e) gegebenenfalls weitere Metallionen von Metallen der zweiten Hauptgruppe, aus den Gruppen der Lanthaniden und/oder Aktiniden und/oder von Metallen mit Ordnungszahlen von 25 bis 30

enthält.

2. Verfahren nach Anspruch 1, dadurch gekennzeichnet, dass man als Alkohol einen einwertigen aliphatischen Alkohol mit 1 bis 6 C-Atomen einsetzt.

3. Verfahren nach Ansprüchen 1 und 2, dadurch gekennzeichnet, dass man Alkohol in bezug auf das Katalysatorsystem in einem Gewichtsverhältnis von 5 : 1 bis 300 : 1 einsetzt.

4. Verfahren nach Ansprüchen 1 bis 3, dadurch gekennzeichnet, dass man als Katalysatorkomponenten a) und b) kationisches Kupfer mit einem oder mehreren Oxy-, Hydroxy- oder anderen Sauerstoff enthaltenden Anionen einsetzt.

5. Verfahren nach Ansprüchen 1 bis 4, dadurch gekennzeichnet, dass als Katalysatorkomponente c) Chlorid verwendet wird.

6. Verfahren nach Anspruch 5, dadurch gekennzeichnet, dass ein Teil des Chlorids in Form eines Ammoniumchlorids und der Rest als Chlorid mit den Gegenionen entsprechend Katalysatorkomponente a) und/oder e) eingesetzt wird.

7. Verfahren nach Ansprüchen 1 bis 6, dadurch gekennzeichnet, dass die Katalysatorkomponente d) eine Stickstoffbase mit 1 bis 700 C-Atomen ist.

8. Verfahren nach Ansprüchen 1 bis 7, dadurch gekennzeichnet, dass im Katalysatorsystem das Verhältnis von Halogenid zu Gesamtmetall, ausgedrückt in Gramm-Atom, 0,5 : 1 bis 2 : 1 und das molare Verhältnis der Stickstoffbase zum Gesamtmetall 0,1 : 1 bis 1,4 : 1 beträgt.

9. Verfahren nach Ansprüchen 1 bis 8, dadurch gekennzeichnet, dass man den Katalysator wiederverwendet, nachdem man einen Anteil von 5 bis 30 Gew.-% entnommen und das darin gebundene Reaktionswasser entfernt hat.

10. Verfahren nach Ansprüchen 1 bis 9, dadurch gekennzeichnet, dass man zwischen 50 und 250 °C und bei weniger als 250 bar arbeitet.

**Claims**

1. Process for the preparation of carbonic acid esters from alcohols, carbon monoxide and molecular oxygen in the liquid phase, characterised in that it is carried out in the presence of a catalyst system which contains a) copper and/or copper ions, b) oxygen anions and/or anions containing oxygen, c) halide ions, d) one or more nitrogen bases and e) optionally, other metal ions of metals from main group 2, of the lanthanide and/or actinide groups and/or of metals with atomic numbers of 25 to 30.

2. Process according to Claim 1, characterised in that a monohydric aliphatic alcohol with 1 to 6 C atoms is employed as the alcohol.

3. Process according to Claims 1 and 2, characterised in that the alcohol is employed in a weight ratio of 5 : 1 to 300 : 1, relative to the catalyst system.

4. Process according to Claims 1 to 3, characterised in that cationic copper together with one or more oxy-anions, hydroxy-anions or other oxygen-containing anions is employed as catalyst components a) and b).

5. Process according to Claims 1 to 4, characterised in that a chloride is used as catalyst component c).

6. Process according to Claim 5, characterised in that some of the chloride is employed in the form of an ammonium chloride and the remainder is employed as a chloride together with ions of opposite charge which correspond to catalyst components a) and/or e).

7. Process according to Claims 1 to 6, characterised in that catalyst component d) is a nitrogen base with 1 to 700 C atoms.

8. Process according to Claims 1 to 7, characterised in that, in the catalyst system, the ratio of halide to total metal, expressed in gram-atoms, is 0.5 : 1 to 2 : 1 and the molar ratio of nitrogen base to total metal is 0.1 : 1 to 1.4 : 1.

9. Process according to Claims 1 to 8, characterised in that the catalyst is re-used, after a proportion of 5 to 30% by weight has been separated off and the water of reaction bonded therein has been removed.

10. Process according to Claims 1 to 9, characterised in that it is carried out at between 50 and 250 °C and under less than 250 bars.

**Revendications**

1. Procédé de préparation d'esters de l'acide carbonique à partir d'alcools, du monoxyde de carbone et de l'oxygène moléculaire en phase liquide, procédé caractérisé en ce qu'on travaille en présence d'un système de catalyseur qui contient:

a) du cuivre et/ou des ions cuivre;
b) des anions oxygène et/ou des anions contenant de l'oxygène;
c) des ions halogénure;
d) une ou plusieurs bases azotées;
e) éventuellement d'autres ions de métaux du second groupe principal, des groupes ou familles de lanthanides et/ou des actinides et/ou de métaux dont les numéros atomiques vont de 25 à 30.

2. Procédé selon la revendication 1, caractérisé en ce qu'on utilise comme alcool un monoalcool aliphatique comportant 1 à 6 atomes de carbone.

3. Procédé selon les revendications 1 et 2, caractérisé en ce qu'on utilise l'alcool, par rapport au système du catalyseur, selon un rapport pondéral de 5 : 1 à 300 : 1.

4. Procédé selon les revendications 1 à 3, caractérisé en ce qu'on utilise, comme composants a) et b) du catalyseur, du cuivre cationique avec un ou plusieurs anions contenant une fonction oxy, hydroxyde ou d'autres anions contenant de l'oxygène.

5. Procédé selon les revendications 1 à 4, caractérisé en ce qu'on utilise comme composant c) du catalyseur du chlorure.

6. Procédé selon la revendication 5, caractérisé en ce qu'on utilise une partie du chlorure sous forme d'un chlorure d'ammonium et le reste sous forme de chlorure avec les ions antagonistes correspondant au(x) composant(s) a) et/ou e) du catalyseur.

7. Procédé selon les revendications 1 à 6, caractérisé en ce que le composant d) du catalyseur est une base azotée comportant 1 à 700 atomes de carbone.

8. Procédé selon les revendications 1 à 7, caractérisé en ce que, dans le système du catalyseur, le rapport de l'halogénure au métal total, exprimé en atomes-grammes, est de 0,5 : 1 à 2 : 1, et le rapport molaire de la base azotée au métal total est de 0,1 : 1 à 1,4 : 1.

9. Procédé selon les revendications 1 à 8, caractérisé en ce qu'on utilise à nouveau le catalyseur, après avoir retiré une partie représentant 5 à 30% en poids et en avoir éliminé l'eau de réaction qui y était liée.

10. Procédé selon les revendications 1 à 9, caractérisé en ce qu'on travaille entre 50 et 250 °C et sous une pression inférieure à 250 bar.